# EUROPEAN PATENT SPECIFICATION

(11) **EP 1 874 729 B1**
(45) Date of publication and mention of the grant of the patent: **16.11.2011**
(21) Application number: 06749890.7
(22) Date of filing: 11.04.2006
(51) Int. Cl.: A61L 27/18, A61L 27/34, A61L 29/06, A61L 31/06, A61L 31/10, C07F 9/572, C08G 79/02, A61K 47/34

(54) **BIODEGRADABLE POLYPHOSPHAZENES CONTAINING PYRROLIDONE SIDE GROUPS**
BIOLOGISCH ABBAUBARE POLYPHOSPHAZENE MIT PYRROLIDONSEITENGRUPPEN
POLYPHOSPHAZENES BIODEGRADABLES CONTENANT DES GROUPES PYRROLIDONE LATERAUX

(30) Priority: 15.04.2005 US 672191 P
(43) Date of publication of application: 09.01.2008
(73) Proprietor: Parallel Solutions, Inc., Cambridge, Massachusetts 02138 (US)
(72) Inventor: ANDRIANOV, Alexander, K., Belmont, Massachusetts 02478 (US); MARIN, Alexander, Newton, Massachusetts 02466 (US)
(74) Representative: Maggs, Michael Norman
(86) International application number: PCT/US2006/013661
(87) International publication number: WO 2006/113274

(56) References cited:
- WO-A1-01/36516
- WO-A1-88/09664
- PREDVODITELEV D A ET AL: "Synthesis of (P-N)-amino-acid derivatives of glycerophosphorylethanolamine" JOURNAL OF ORGANIC CHEMISTRY OF THE USSR, vol. 11, no. 6, June 1975 (1975-06), pages 1180-1184, XP9136166 ISSN: 0022-3271
- DATABASE CAPLUS [Online] PREDVODITELEV D.A. ET AL.: 'Synthesis of N-amino acid derivatives of glycerylphosphorylethanolamine', XP003001975 Retrieved from STN Database accession no. (1975:593663) & ZHURNAL ORGANICHESKOI KHIMII vol. 11, no. 6, 1975, pages 1190 - 1195

## Description

### Biodegradable Polyphosphazenes Containing Pyrrolidone Side Groups

WO 01/36516 relates to biodegradable polyphosphazenes represented by Formula 1 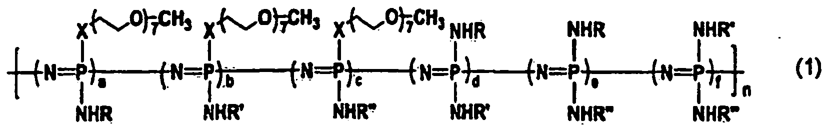 and a preparation method thereof. These polyphosphazenes are said to not only have thermosensitivity and biodegradability, but also their phase transition temperature and degradation rate can be controlled.

WO 88/09664 discloses that a biologically active substance, e.g. a therapeutic agent, is delivered from an inorganic polymeric system including a polyphosphazene polymer body, the active substance being physically entrapped by the polymer body. When the system is exposed to an aqueous fluid, e.g. when implanted in physiological fluid of a living mammal, the agent is released from the polymer body at a sustained and controlled rate.

### BRIEF DESCRIPTION OF THE SEVERAL VIEWS OF THE DRAWINGS

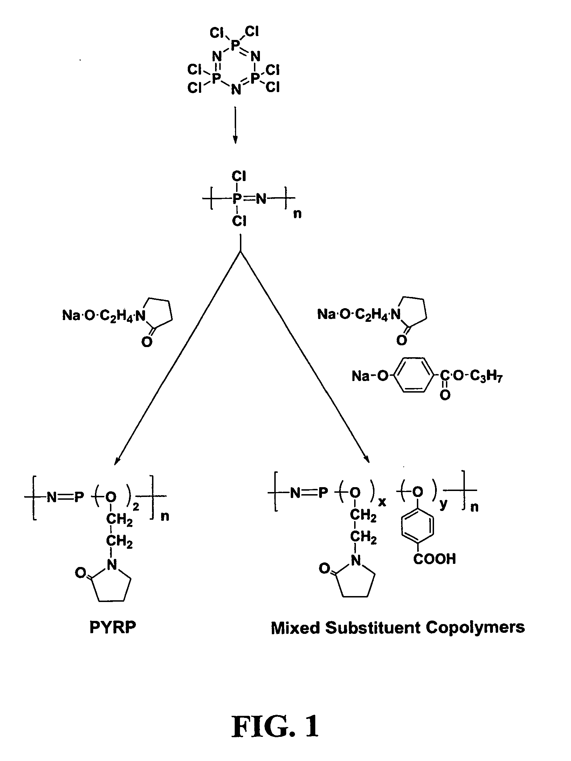

Fig. 1 represents a synthetic pathway to polyphosphazenes with N-alkyl pyrrolidone side groups.
Fig. 2 represents molecular weight loss of the PYRP in aqueous solutions at various pH versus time (weight average molecular weight determined by light scattering, 55°C, 1 mg/mL).
Fig. 3 represents molecular weight loss of the PYRP in aqueous solutions at various pH versus
time (weight average molecular weight determined by GPC with PEO standards, 55°C, 1 mg/mL).
Fig. 4 represents molecular weight loss of the PYRP (1), CP1 (2), CP2 (3), and PCPP (4) in aqueous solutions versus time (weight average molecular weight determined by light scattering, 55°C, 1 mg/mL).
Fig. 5 represents release of p-hydroxybenzoic acid in aqueous solutions for CP1 (1) and PCPP (2) versus time (55°C, 1 mg/mL).
Fig. 6 represents ³¹p NMR spectra of PYRP in D₂O, pH 6 (a), in D₂O after addition of deuterated hydrochloric acid, pH 2 (b), and after incubation of PYRP in D₂O - deuterated hydrochloric acid, pH 2 at 55 °C for 5 days (c). Molecular weights, determined by light scattering were 370,000 g/mol (a, b) and 27,000 g/mol (c).

### DETAILED DESCRIPTION OF THE INVENTION

This invention relates to polyphosphazenes containing pyrrolidone side groups. Such polymers can be particularly useful as drug delivery carriers, plasma expanders, and biocompatible coatings for medical devices.

Polyphosphazenes are polymers with backbones including alternating phosphorus and nitrogen atoms, separated by alternating single and double bonds. Each phosphorous atom is covalently bonded to two pendant groups ("A").

The repeat unit of a polyphosphazene has the following formula: 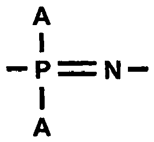 wherein each "A" may be the same, or different, and wherein the unit is repeated "n" times.

When the polyphosphazene has only one type of pendant group or side group repeatedly attached to its backbone the polymer is said to be a homopolymer. When the polyphosphazene has more than one type of pendant group, the polyphosphazene is a copolymer. When the groups vary randomly throughout the polymer, the polyphosphazene is a random copolymer. Phosphorous can be bound to two like groups, or to two different groups.

Polyphosphazenes with desired side groups can be produced by initially producing a reactive macromolecular precursor - poly(dichlorophosphazene) and then by reacting it with a nucleophile that contains the desired side group, such nucleophiles include alcohols, amines, or thiols. Polyphosphazenes with two or more types of pendant groups can be produced by reacting poly(dichlorophosphazene) with two or more types of nucleophiles in a desired ratio. Nucleophiles can be added to the reaction mixture simultaneously or in sequential order. The resulting ratio of pendant groups in the polyphosphazene will be determined by a number of factors, including the ratio of starting materials used to produce the polymer, the order of addition, the temperature at which the nucleophilic substitution reaction is carried out, and the solvent system used. The ratio of groups in the polymer can be easily determined by one skilled in the art.

The polymers of the present invention may be produced by reacting poly(dichlorophosphazene) with an organic nucleophile containing a pyrrolidone moiety. For example, an organic compound containing hydroxyl group and N-alkyl pyrrolidone may be reacted with the reactive chlorine atoms on poly(dichlorophosphazene). One or a mixture of organic compounds can be used to result in a homopolymer or mixed substituent copolymers correspondingly. Hydroxyl group of the organic compound can be activated with sodium, sodium hydride, or sodium hydroxide by procedures known in the art and then reacted with chlorine atoms attached to the polyphosphazene backbone.

Thus, in one aspect, the present invention provides a polyphosphazene polymer that contains repeating units of the following formula: 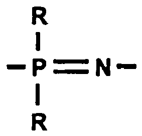 wherein in each monomeric unit of the polymer, each R is the same or different, and wherein at least a portion of the monomeric units of the polymer one or more of the R groups is "X." Wherein "X" is: 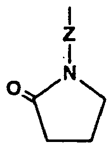 wherein Z is oxyethyl: 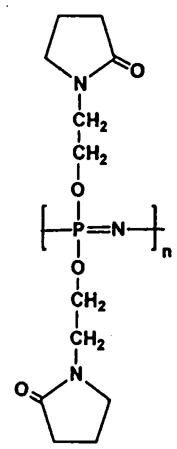 or wherein Z is oxyethyl and at least a portion of the R side groups are carboxylatophenoxy groups:

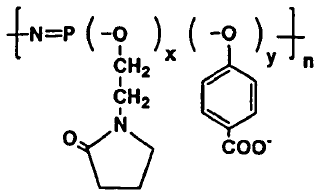

The remaining R groups may be one or more of a wide variety of substituent groups. As representative, non-limiting examples of such groups there may be mentioned: aliphatic; aryl; aralkyl; alkaryl; carboxylic acid; heteroaromatic; carbohydrates, including glucose; heteroalkyl; halogen; (aliphatic)amino- including alkylamino-; heteroaralkyl; di(aliphatic)amino- including dialkylamino-, arylamino-, diarylamino-, alkylarylamino-; - oxyaryl including but not limited to -oxyphenylCO₂H, -oxyphenylSO₃H, -oxyphenylhydroxyl and -oxyphenylPO₃H; -oxyaliphatic including -oxyalkyl, -oxy(aliphatic)CO₂H, - oxy(aliphatic)SO₃H, -oxy(aliphatic)PO₃H, and -oxy(aliphatic)hydroxyl, including oxy(alkyl)hydroxyl; -oxyalkaryl, -oxyaralkyl; -thioaryl; aminoacid, aminoacid ester, thioaliphatic including -thioalkyl; -thioalkaryl; thioaralkyl; -NHC(O)O-(aryl or aliphatic);O--[(CH₂)ₓO]_{y}--CH₂)--O--[(CH₂)ₓO]_{y}(CH₂)ₓNH(CH₂)ₓSO₃H; and --O--[(CH₂)ₓO]_{y}-(aryl or aliphatic); wherein x is 1-8 and y is an integer of 1 to 20. The remaining R groups can be immunostimulating carboxylic acid containing groups. The groups can be bonded to the phosphorous atom through, for example, an oxygen, sulfur, nitrogen, or carbon atom.

It is an aspect of the instant invention that the polyphosphazene polymer as hereinabove described and as hereinbelow described has an overall molecular weight of 1,000 g/mol. to 10,000,000 g./mol.

Polyphosphazenes of the present invention can be homopolymers, having one type of side groups, or mixed substituent copolymers, having two or more types of side groups.

Polymers of the present invention contain N-ethyl pyrrolidone group:

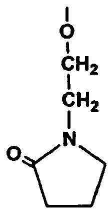

A polymer of the present invention is poly{di[2-(2-oxo-1-pyrrolidinyl)ethoxy]phosphazene}or poly{di[2-(1-pyrrolid-2-one)ethoxy]phosphazene}, **PYRP:** 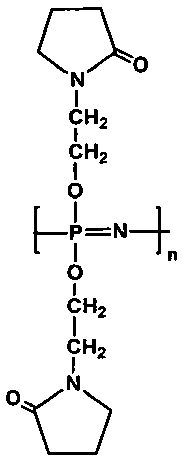 wherein n is a number that provides the desired molecular weight. For example, n may be from 10 to 10,000.

In mixed substituent copolymers there is at least one type of side group that contains pyrrolidone functionality and one type of side groups that does not contain pyrrolidone functionality. Side groups that do not contain pyrrolidone functionalities can be introduced in a polyphosphazene copolymer to modulate physical or physico-chemical properties of the polymer. Such side groups can be used, for example, to modulate water-solubility, biodegradability, hydrophobicity, or to make them biologically active. As non-limiting examples of such non-pyrrolidone functionality containing physical or physico-chemical property modulating side groups there may be mentioned side groups that include amino acids and their esters, carboxylic acids, phenoxy, alkoxy, hydroxy, halogen, and methoxyethoxyethoxy.

In a co-polymer, in general, the side groups that contain a pyrrolidone functionality comprise from 0.5% to 99.5% of the total side groups and preferably at least 10% of the total side groups.

An example of mixed substituent copolymers is the following:

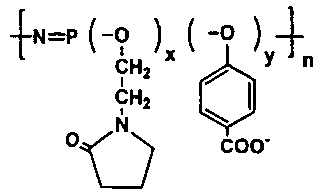

The polyphosphazenes of the present invention are polymers that are preferably biodegradable when administered to either humans or animals. Biodegradability of the polymer prevents eventual deposition and accumulation of polymer molecules at distant sites in the body, such as the spleen. The term biodegradable, as used herein, means a polymer that degrades within a period that is acceptable in the desired application, typically less than about five years and most preferably less than about one year.

The polyphosphazenes of the present invention are polymers that are preferably biocompatible. "Biocompatible material" may be defined as a polymer that is designed and constructed to be placed in or onto the body or to contact fluid or tissue of the body. Ideally, a biocompatible polymer will not induce undesirable reactions in the body such as blood clotting, tissue death, tumor formation, allergic reaction, foreign body reaction (rejection) or inflammatory reaction; will have the physical or biological properties required to function for the intended purpose; can be purified, fabricated, and sterilized easily; and will substantially maintain its function during the time that it remains in contact with the body.

The polyphosphazenes of the present inventions can be used in the water-soluble form or as solid materials.

They may be used in controlled release formulations and can be dissolved and/or suspended in water or any other suitable liquid medium in which the polymers are soluble and/or dispersible and combined with a pharmacological or pharmaceutical compound or composition to form a composition of matter. The polymer will act as a matrix for the pharmaceutical to provide an article of manufacture for the controlled release of such pharmaceutical. It can stabilize and protect the pharmaceutical. The pharmaceuticals can be attached to the polymer covalently or they can be associated with the polymer through noncovalent linkages, such as ionic, hydrogen bonds, or hydrophobic interactions.

The polymers may be used for the encapsulation of pharmaceutical agents to produce microspheres, microcapsules, micelles, or the polymers may be used to stabilize liposomes.

Pharmaceuticals which may be included in the resulting article of manufacture are listed in the Physicians' Desk Reference, 57th Edition (2003), and include allergens, amebicides and trichomonacides, amino acid preparations, analeptic agents, analgesics, analgesics/antacids, anesthetics, anorexics, antacids, antihelmintics, antialcohol preparations, antiarthritics, antiasthma agents, antibacterials and antiseptics, antibiotics, antiviral antibiotics, anticancer preparations, anticholinergic drug inhibitors, anticoagulants, anticonvulsants, antidepressants, antidiabetic agents, antidiarrheals, antidiuretics, antienuresis agents, antifibrinolytic agents, antifibrotics (systemic), antiflatulents, antifungal agents, antigonadotropin, antihistamines, antihyperammonia agents, anti-inflammatory agents, antimalarials, antimetabolites, antimigraine preparations, antinauseants, antineoplastics, anti-obesity preparations, antiparasitics, anti-parkinsonism drugs, antipruritics, antipyretics, antispasmodics and antichloinergics, antitoxoplasmosis agents, antitussives, antivertigo agents, antiviral agents, biologicals, bismuth preparations, bone metabolism regulators, bowel evacuants, bronchial dilators, calcium preparations, cardiovascular preparations, central nervous system stimulants, cerumenolytics, chelating agents, choleretics, cholesterol reducers and anti-hyperlipemics, colonic content acidifiers, cough and cold preparations, decongestants, expectorants and combinations, diuretics, emetics, enzymes and digestants, fertility agents, fluorine preparations, galactokinetic agents, geriatrics, germicides, hematinics, hemorrhoidal preparations, histamine H. receptor antagonists, hormones, hydrocholeretics, hyperglycemic agents, hypnotics, immunosuppressives, laxatives, mucolytics, muscle relaxants, narcotic antagonists, narcotic detoxification agents, ophthalmological osmotic dehydrating agents, otic preparations, oxytocics, parashypatholytics, parathyroid preparations, pediculicides, phosphorus preparations, premenstrual therapeutics, psychostimulants, quinidines, radiopharmaceuticals, respiratory stimulants, salt substitutes, scabicides, sclerosing agents, sedatives, sympatholytics, sympathomimetics, thrombolytics, thyroid preparations, tranquilizers, tuberculosis preparations, uricosuric agents, urinary acidifiers, urinary alkalinizing agents, urinary tract analgesic, urological irrigants, uterine contractants, vaginal therapeutics and vitamins.

The polymers of the present invention are used in an amount sufficient to form a matrix around the composition or material to be released in the time release formulations or to act as a carrier for such compositions or materials in such formulations and can be employed in an amount sufficient to permit the delayed time release of a composition or material into its environment, e.g., anywhere from about 1% by weight to about 99% by weight of the time release formulation and preferably from about 5% by weight to about 99% by weight of the time release formulation.

Polymers of the present invention can be used as coatings or materials for "medical devices" - articles that have surfaces that contact blood or other bodily tissues in the course of their operation. This can include, for example, extracorporeal devices for use in surgery such as blood oxygenators, blood pumps, blood sensors, tubing used to carry blood, and the like, which contact blood, which is then returned to the patient. This can also include implantable devices such as vascular grafts, stents, electrical stimulation leads, heart valves, orthopedic devices, catheters, guide wires, shunts, sensors, replacement devices for nucleus pulposus, cochlear or middle ear implants, intraocular lenses, and the like. Coatings or materials for medical devices can be designed to release drugs for an extended period of time.

The polyphosphazene polymers of the present invention may be used as coating agents for the construction of monolayer and multilayer assemblies. Such assemblies are constructed by layer-by-layer polyelectrolyte deposition. This includes direct adsorption of polyelectrolytes onto suitable solids, at the interface between the solid and a fluid phase containing the monolayer forming molecules. The process can be continued until the targeted number of monolayers, one upon the other, is obtained (US Patent 4,539,061). Typically, a multilayer coating can contain 5 to 20 layers of polyelectrolytic materials. Desired functional groups can be included at the upper surface of the coating to obtain a surface with a required set of properties. Multilayer coatings can be used in a wide range of industrial applications, such as but not limited to, preparation of artificial membranes, fabrication of passive and active ultrathin film components for novel microelectronic and optical devices, ultrathin photoresists, and molecular films useful in solar energy conversion, etc. Multilayer coatings can be used in biomedical devices. Biomedical devices include a wide variety of devices used in the biological, medical, or personal care industries and include, but are not limited to, ophthalmic lenses, drug delivery devices such as oral osmotic devices and transdermal devices, catheters, contact lens disinfection and cleaning containers, breast implants, stents, intervertebral discs, artificial organs and tissues, and the like. Many devices and materials used in biomedical applications require certain properties in the bulk of the device or material with distinct and separate properties required for the surface. For example, a contact lens may have a core or bulk material which is highly oxygen permeable and hydrophobic, and a surface which has been treated or coated to increase the hydrophilicity, thereby allowing the lens to move freely on the eye. Other biomaterials require coatings effective in preventing protein adsorption on biosurfaces and preventing fouling.

The invention now will be described with respect to the following examples; it is to be understood, however, that the scope of the present invention is not intended to be limited thereby.

### Example 1. Synthesis of Poly{di[2-(2-oxo-1-pyrrolidinyl)ethoxy]phosphazene}, PYRP.

A suspension of sodium hydride (0.066 g; 0.0026 mol) in 1,4-dioxane (0.0008 L) was slowly added to 1-(2-hydroxyethyl) pyrrolidone (1.59 g; 0.0123 mol) in 1,4-dioxane (0.010 L) under nitrogen to form sodium 1-(2-hydroxyethyl) pyrrolidone. This solution was diluted with 0.015 L of 1,4-dioxane, and then heated to 50 °C with stirring under dry nitrogen. 0.002 L of polydichlorophosphazene (0.116 g; 0.001 moles) was then added slowly via syringe over a period of five minutes. The reaction mixture was stirred for fifteen hours at 50 °C, then cooled to ambient temperature and precipitated with hexane. The precipitate was collected by decantation, dried under vacuum, and then dissolved in water. The polymer then was purified using a Biocad Perfusion Chromatography Workstation (Applied Biosystems, Foster Hills, CA) equipped with a Modcol CER 3662 column using 0.02 M ammonium bicarbonate. Polymer fractions were collected and then lyophilized. The yield was 0.113 g (37.5 % of theoretical). Polymer structure was confirmed by ¹H NMR, ¹³C NMR and ³¹P NMR (Table 1) and molecular weight was determined by GPC-light scattering and GPC using poly(ethylene oxide) standards (Table 2).

**Table 1. NMR characterization data.**

| Polymer | NMR, ppm | |
|---|---|---|
| 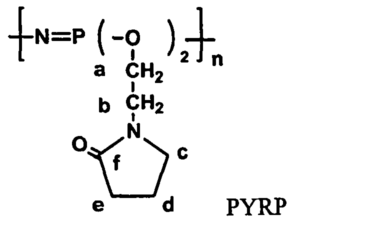 | ¹H | 2.02 (**d**); 2.38 (**e**); 3.49 (**b**); 3.51 (**c**); 4.03 (**a**) |
| | ¹³C | 178.11 (**f**); 63.65 (**a**); 48.61 (**b**); 43.11 (**c**); 30.79 (**e**); 17.78 (**d**) |
| | ³¹P | -7.4 |
| 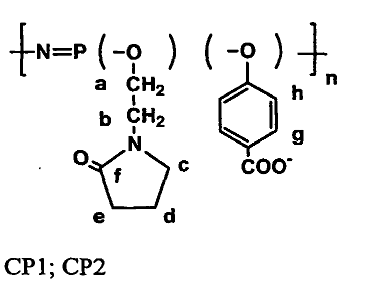 | ¹H | 1.97 (**d**); 2.37 (**e**); 3.41 (**b**); 3.47 (**c**); 3.95 (**a**); 7.25 (**h**); 7.94 (**g**) |
| | ³¹P | -19.7; -13.7; -7.4. |

**Table 2. Polymer compositions and molecular weights.**

| Polymer | 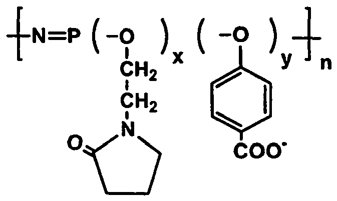 | | | | |
|---|---|---|---|---|---|
| | Composition, x : y | | | Mw, g/mol x 10⁻³ | |
| | Expected * | ¹H NMR | UV-HPLC | MALLS-GPC ^{#} | GPC (PEO) ^{Δ} |
| PYRP | 2:0 | 2:0 | 2:0 | 370 | 305 |
| CP1 | 1.70 : 0.30 | 1.70 : 0.30 | 1.64 : 0.36 | 1000 | 610 |
| CP2 | 0.3 : 1.70 | 0.18 : 1.82 | 0.18 : 1.82 | 513 | 471 |

| | | | | | |
|---|---|---|---|---|---|
| * based on the composition of the reaction mixture ^{#} based on GPC with multi angle laser light scattering detection ^{Δ} based on GPC with PEO standards | | | | | |

### Example 2. Synthesis of Poly{[2-(2-oxo-1-pyrrolidinyl)ethoxy](4-carboxylatophenoxy)phosphazene} (CP1).

A suspension of sodium hydride (0.086 g; 0.003 moles) in 1,4-dioxane (0.002 L) was slowly added to a solution of 1-(2-hydroxyethyl) pyrrolidone (2.08 g; 0.016 moles) in 0.010 L of 1,4-dioxane under nitrogen to form sodium 1-(2-hydroxyethyl) pyrrolidone. Sodium propyl 4-hydroxybenzoate was prepared by adding a suspension of sodium hydride (0.61 g; 0.025 moles) in diglyme (0.004 L) to a solution of propyl ester of 4-hydroxybenzoic acid (5.19 g; 0.029 moles) in 0.010 L diglyme under dry nitrogen. 0.0005 L of the sodium propyl 4-hydroxybenzoate solution was added to the sodium 1-(2-hydroxyethyl) pyrrolidone solution at ambient temperature.

0.002 L of polydichlorophosphazene (0.116 g; 0.001 moles) was then added slowly at room temperature over a period of five minutes. Following the addition, the temperature was raised to 50 °C, and stirred for fifteen hours. 0.001 L of 12.7 N aqueous potassium hydroxide solution was slowly added, and the reaction mixture was stirred for one hour at 50 °C.

The precipitate was collected by decantation, dried and dissolved in water. The polymer then was purified chromatographically as described above. The yield of polymer (CP1) was 0.228 g (75 % of theoretical). Polymer characterization data is presented in Tables 1 and 2.

The composition of mixed substituent polymer was determined using two methods. (1) It was calculated based on the ratio between the peak areas of ethylene protons of the ethylpyrrolidone side group and the aromatic protons of the carboxylatophenoxy side group in ¹H NMR. (2) The composition was established using HPLC based on the differences in the UV absorbance of PCPP and PYRP at 254 nm in PBS (pH 7.4). Calibration curves were obtained for the mixtures of PCPP and PYRP by plotting HPLC peak areas at 254 nm versus mixture composition. The total polymer concentration was maintained at 1 mg/mL and the results were processed using Millenium (Waters, Milford, MA) software. A copolymer was then analyzed by HPLC using the same conditions and its molar composition was determined using calibration curves obtained for the mixtures of homopolymers. Polymer composition data is presented in Table 2.

### Example 3. Synthesis of Poly{[2-(2-oxo-1-pyrrolidinyl)ethoxy](4-carboxylatophenoxy)phosphazene} (CP2).

A suspension of sodium hydride (0.247 g; 0.0098 moles) in 1,4-dioxane (0.105 L) was slowly added to 1-(2-hydroxyethyl)-2-pyrrolidone (5.989 g; 0.0464 moles) in 1,4-dioxane (0.025 L) under a dry nitrogen to form a sodium 1-(2-hydroxyethyl)-2-pyrrolidone solution. Sodium propyl 4-hydroxybenzoate was prepared by adding a suspension of sodium hydride (0.707 g; 0.0279 moles) in diglyme (0.003 L) to 4-hydroxybenzoate propyl ester (6.055 g; 0.3360 moles) in diglyme (0.010 L) under a dry nitrogen atmosphere. 0.003 L polydichlorophosphazene (0.232 g; 0.002 moles) was diluted with 0.013 L diglyme at room temperature under nitrogen and then heated to 50 °C. 0.0024 L of the sodium propyl 4-hydroxybenzoate was added while stirring. The temperature was increased to 100 °C, the reaction mixture was stirred for three hours and then cooled to 50 °C. To this 0.015 L of the sodium 1-(2-hydroxyethyl)-2-pyrrolidone solution was slowly added and the reaction continued for twenty hours. 0.010 L of 12.7 N potassium hydroxide solution was slowly added, and stirred for one hour at 50 °C. The precipitated polymer was collected, dissolved in distilled water, and precipitated by adding 1 N hydrochloric acid until pH 3. The precipitate was re-dissolved in 0.05 M of ammonium bicarbonate and purified by preparative HPLC as described above. The yield of polymer (CP2) was 0.29 g (38.6 % of theoretical). Polymer composition was determined as described in Example 2. Characterization data is presented in Tables 1 and 2.

### Example 4. Polymer Degradation Studies.

Solutions of polymers PYRP, CP1, CP2, and PCPP were prepared at a concentration of 1 mg/mL in the following buffers: citrate buffer, pH 3.0 (0.040 M citric acid; 0.021M sodium hydroxide; 0.060 M sodium chloride), Tris buffer, pH 7.4 (0.020 M Tris, 0.9% NaCl) and borate buffer, pH 9.3 (0.02 M sodium tetraborate). Samples were incubated at room temperature for 1 hour upon shaking to assure complete dissolution and then filtered using 0.45 µm Millex-HV syringe filters (Millipore, Bedford, MA).

Degradation studies were performed at 55 °C. Vials containing polymer solutions were incubated in a G24 Environmental Incubator Shaker (New Brunswick Scientific, Edison, NJ). 0.2 mL samples were collected periodically for the determination of molecular weight and degradation products. Analysis was conducted using size exclusion HPLC with triple detection system - multi-angle laser light scattering (DAWN DSP-F Wyatt Technology, Santa Barbara, CA), Waters 996 photo-diode array, and Waters 410 refractive index (Waters, Milford, MA). Absolute molecular weight parameters were determined using light scattering detection, refractive index detector as a mass detector, and ASTRA 2.1 software (Wyatt Technology, Santa Barbara, CA). Relative molecular weight characteristics were calculated based on calibration curves for poly(ethylene oxide) standards using photo-diode array detection with absorbance measured at 230 nm. Concentration of hydroxybenzoic acid (HBA) was determined by HPLC with UV detection at 280 nm using Millenium (Waters, Milford, MA) software. Phosphate buffered saline PBS, pH 7.4 was used as a mobile phase with a flow rate of 0.75 ml/min and injection volume of 0.1 mL.

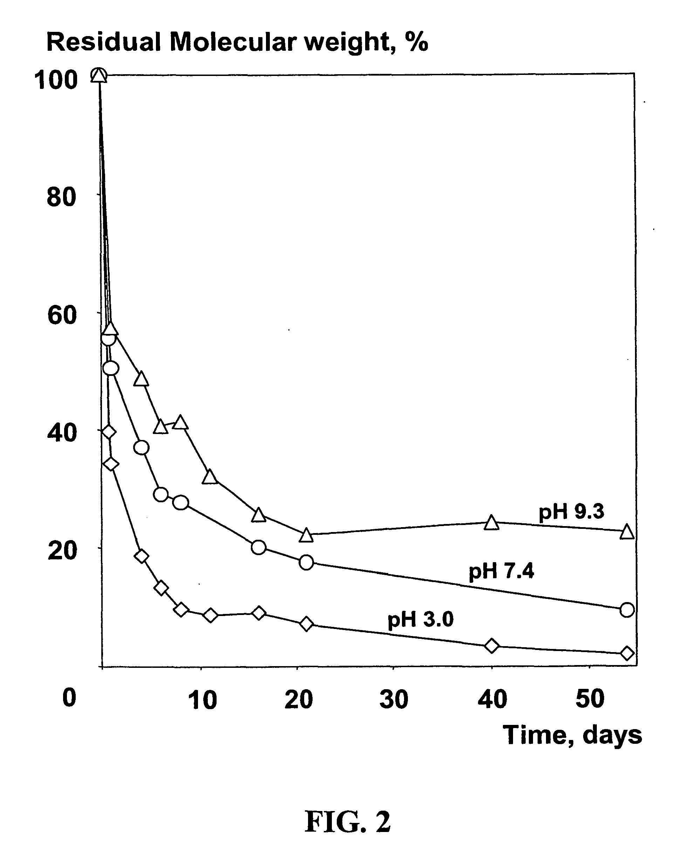

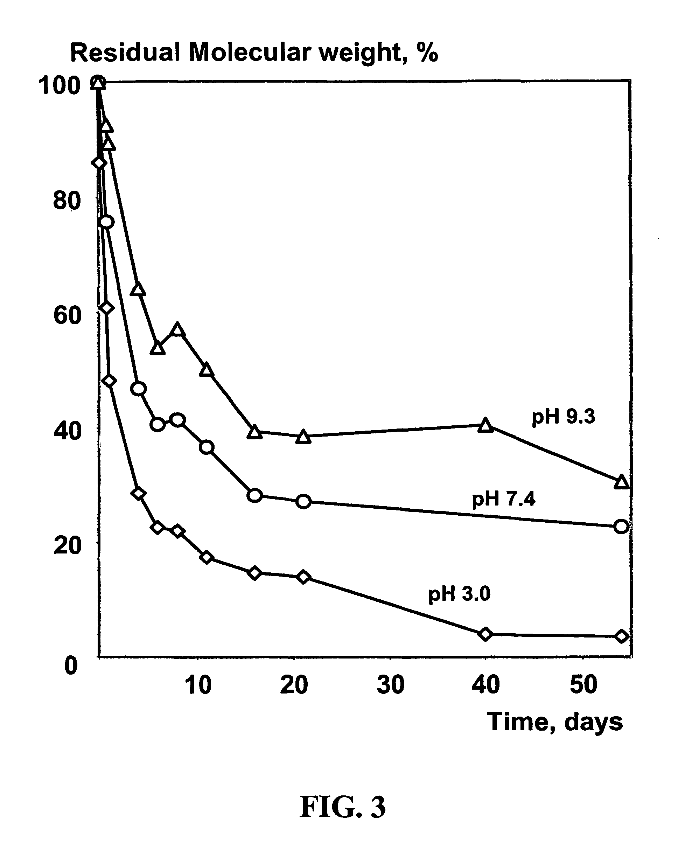

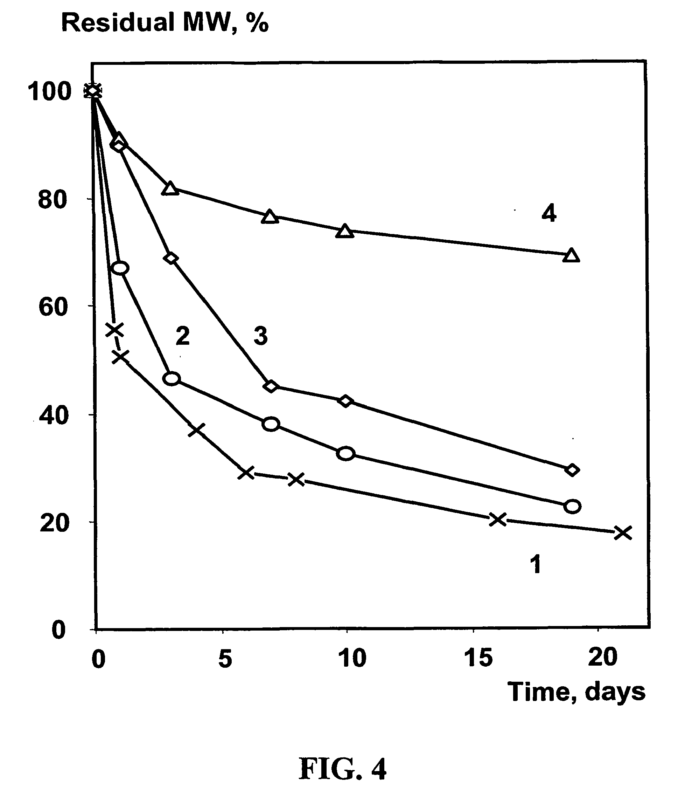

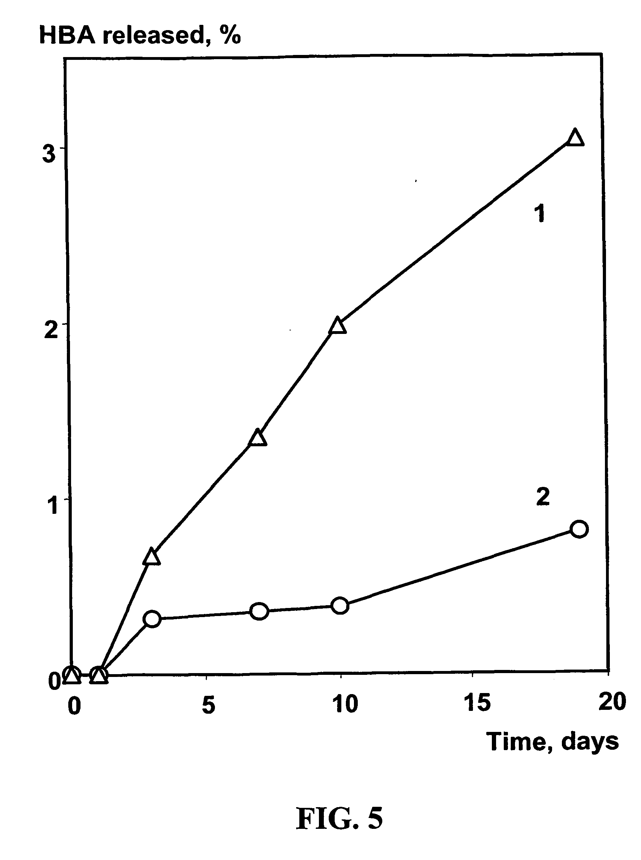

Figures 2 - 3 show the kinetics of weight average molecular weight decrease of PYRP in aqueous solutions with pH 9.3, pH 7.4, and pH 3.0 at 55°C. It appears that the rate of hydrolysis is pH dependent with the degradation rate increasing as pH declines. This relationship is observed for both, absolute molecular weight, determined by GPC with multi-angle laser light scattering detector (Figure 2), and relative molecular weight, measured by GPC using PEO standards (Figure 3). Figure 4 shows degradation profiles for polymers PYRP, CP1, CP2, and PCPP in PBS, pH 7.4. Pyrrolidone containing homopolymer PYRP demonstrated the highest hydrolysis rate, and PCPP the lowest. Introduction of pyrrolidone side groups in the PCPP structure (CP1, CP2) resulted in a pronounced increase in the rate of molecular weight loss. The degradation of PCPP and its copolymer containing pyrrolidone groups (CP1) was also accompanied with a side group cleavage, manifested in the release of hydroxybenzoic acid (Figure 5). Interestingly, the rate of release was higher for a copolymer CP1, which confirms a destabilizing effect of pyrrolidone side groups. Therefore, the ethylpyrrolidone moiety modulated polyphosphazene degradation.

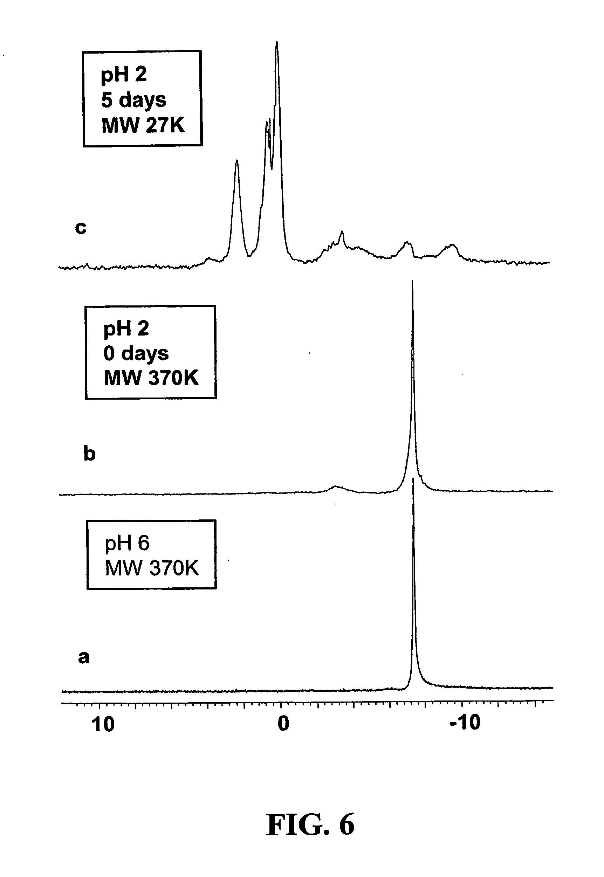

Fig. 6 shows changes in the ³¹P NMR spectra observed during the hydrolysis of polymer PYRP in the acidic aqueous environment. Reaction of the polymer with water under acidic conditions proceeded rapidly enough so that additional peaks appeared immediately after admixing of the hydrochloric acid (Fig. 6b). Peak at -3.2 ppm can be ascribed to PPyrO; signals and -9.8, -7.8 and -8.0 can be assigned to α, β and γ PyrO-P-OPyr. Dramatic change in the ratio between the initial polymer peak PyrO-P-OPyr at -7.4 and α PyrO-P-OPyr peak, as the hydrolysis proceeds, supports this assumption. Peaks in the range of -1 to 4 ppm can be probably associated with low molecular weight phosphates, oligomers, and products of geminal hydrolysis.

¹H NMR analysis of the polymer hydrolyzed in the acidic solution for 5 days still showed original peaks macromolecular peaks of PYRP, which was expected since light scattering analysis demonstrated the presence of polymer with the molecular weight of 27,000 g/mol. In addition N-ethyl pyrrolidone peaks were present, indicating the release of the side group from the polymer. The amount of the low molecular weight compound in the system was estimated to be approximately 70 % of the total of N-alkyl pyrrolidone that can be released from the polymer. No other compounds were detected in the system demonstrating the absence of ring opening reactions.

## Claims

1. A polyphosphazene polymer comprising the following units: 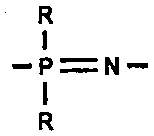 wherein at least one R has the formula: 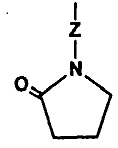 wherein Z is oxyethyl: 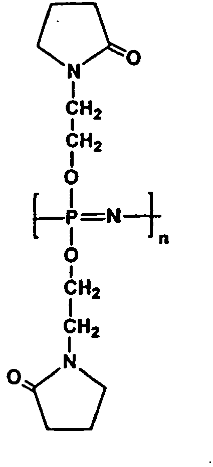 or wherein Z is oxyethyl and at least a portion of the R side groups are carboxylatophenoxy groups:

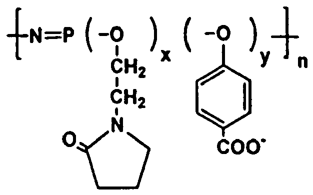

2. The polyphosphazene polymer of claim 1, wherein at least one R group is selected from the group consisting of: aliphatic; aryl; aralkyl; alkaryl; carboxylic acid; heteroaromatic; carbohydrates, including glucose; heteroallcyl; halogen; (aliphatic)amino- including alkylamino-; heteroaralkyl; di(aliphatic)amino- including dialkylamino-, arylamino-, diarylamino-, alkylarylamino-; -oxyaryl including but not limited to -oxyphenylCO₂H, - oxyphenylSO₃H, -oxyphenylhydroxyl and -oxyphenylPO₃H; -oxyaliphatic including - oxyalkyl, -oxy(aliphatic)CO₂H, -oxy(aliphatic)SO₃H, -oxy(aliphatic)PO₃H, and - oxy(aliphatic)hydroxyl, including oxy(alkyl)hydroxyl; -oxyalkaryl, -oxyaralkyl; -thioaryl; aminoacid, aminoacid ester, thioaliphatic including -thioalkyl; -thioalkaryl; thioaralkyl;-NHC(O)O-(aryl or aliphatic); --O--[(CH₂)ₓO]_{y}-CH₂)--O--[(CH₂)ₓOly(CH₂)ₓNH(CH₂)ₓSO₃H; and --O--[(CH₂)ₓO]_{y}-(aryl or aliphatic); wherein x is 1-8 and y is an integer of 1 to 20.

3. The polyphosphazene polymer of claim 1, having a molecular weight of 1,000 g/mol. to 10,000,000 g./mol.

4. The polyphosphazene polymer of claim 1, in the form of a homopolymer.

5. The polyphosphazene polymer of claim 1, in the form of a copolymer.

6. The polyphosphazene polymer of claim 1, wherein n is from 10 to 10,000.

7. The polyphosphazene copolymer of claim 5, wherein the side groups that contain a pyrrolidone functionality comprise from 0.5% to 99.5% of the total side groups.

8. The polyphosphazene copolymer of claim 7, wherein the side groups that contain a pyrrolidone functionality comprise at least 10% of the total side groups.

9. A pharmaceutical composition comprising a pharmaceutically active agent and a carrier for providing controlled release of said pharmaceutically active agent, said carrier comprising the polyphosphazene polymer of claim 1.

10. The pharmaceutical composition of claim 9, wherein said carrier is in a form selected from the group consisting of: matrices, microspheres, microcapsules, micelles, and stabilized liposomes.

11. The pharmaceutical composition of claim 9, wherein said polyphosphazene polymer comprises about 1% by weight to about 99% by weight of said carrier for providing controlled release of said pharmaceutically active agent.

12. The pharmaceutical composition of claim 11, wherein said polyphosphazene polymer comprises about 5% by weight to about 99% by weight of said carrier for providing controlled release of said pharmaceutically active agent.

13. The pharmaceutical composition of claim 9, wherein said pharmaceutically agent is seleceted from the group consisting of: allergens, amebicides and trichomonacides, amino acid preparations, analeptic agents, analgesics, analgesics/antacids, anesthetics, anorexics, antacids, antihelmintics, antialcohol preparations, antiarthritics, antiasthma agents, antibacterials and antiseptics, antibiotics, antiviral antibiotics, anticancer preparations, anticholinergic drug inhibitors, anticoagulants, anticonvulsants, antidepressants, antidiabetic agents, antidiarrheals, antidiuretics, antienuresis agents, antifibrinolytic agents, antifibrotics (systemic), antiflatulents, antifungal agents, antigonadotropin, antihistamines, antihyperammonia agents, anti-inflammatory agents, antimalarials, antimetabolites, antimigraine preparations, antinauseants, antineoplastics, anti-obesity preparations, antiparasitics, anti-parkinsonism drugs, antipruritics, antipyretics, antispasmodics and antichloinergics, antitoxoplasmosis agents, antitussives, antivertigo agents, antiviral agents, biologicals, bismuth preparations, bone metabolism regulators, bowel evacuants, bronchial dilators, calcium preparations, cardiovascular preparations, central nervous system stimulants, cerumenolytics, chelating agents, choleretics, cholesterol reducers and anti-hyperlipemics, colonic content acidifiers, cough and cold preparations, decongestants, expectorants and combinations, diuretics, emetics, enzymes and digestants, fertility agents, fluorine preparations, galactokinetic agents, geriatrics, germicides, hematinics, hemorrhoidal preparations, histamine H. receptor antagonists, hormones, hydrocholeretics, hyperglycemic agents, hypnotics, immunosuppressives, laxatives, mucolytics, muscle relaxants, narcotic antagonists, narcotic detoxification agents, ophthalmological osmotic dehydrating agents, otic preparations, oxytocics, parashypatholytics, parathyroid preparations, pediculicides, phosphorus preparations, premenstrual therapeutics, psychostimulants, quinidines, radiopharmaceuticals, respiratory stimulants, salt substitutes, scabicides, sclerosing agents, sedatives, sympatholytics, sympathomimetics, thrombolytics, thyroid preparations, tranquilizers, tuberculosis preparations, uricosuric agents, urinary acidifiers, urinary alkalinizing agents, urinary tract analgesic, urological irrigants, uterine contractants, vaginal therapeutics and vitamins.

14. A Poly{di[2-(2-oxo-1-pyrrolidinyl)ethoxy]phosphazene} as depicted by Formula I: 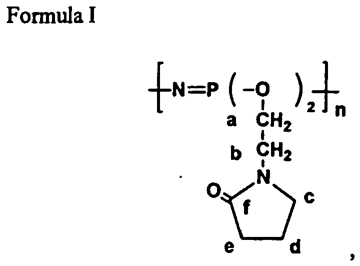 wherein each of a, b, c, d, e, and f are NMR data characterizing said Poly{di[2-(2-oxo-1-pyrrolidinyl)ethoxy]phosphazene} as follows:
| NMR, ppm | |
|---|---|
| ¹H | 2.02 (**d**); 2.38 (**e**); 3.49 (**b**); 3.51 (**c**); 4.03 (**a**) |
| ¹³C | 178.11 (**f**); 63.65 (**a**); 48.61 (**b**); 43.11 (**c**); 30.79 (**e**); 17.78 (**d**) |
| ³¹P | -7.4 |

15. An extracorporeal device for use in surgery, comprising coatings or materials comprising the polyphosphazene polymer of claim 1.

16. The extracorporeal device of claim 15, in a form selected from the group consisting of: blood oxygenators, blood pumps, blood sensors, and tubing used to carry blood.

17. A surgically implantable device, comprising coatings or materials comprising the polyphosphazene polymer of claim 1.

18. The surgically implantable device of claim 17, in a form selected from the group consisting of vascular grafts, stents, electrical stimulation leads, heart valves, orthopedic devices, catheters, guide wires, shunts, sensors, replacement devices for nucleus pulposus, cochlear or middle ear implants, and intraocular lenses.

## Patentansprüche

1. Polyphosphazen-Polymer, welches folgende Einheiten umfasst: 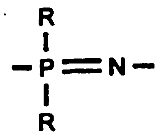 wobei mindestens ein R folgende Formel aufweist: 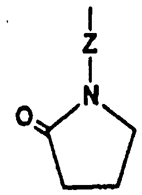 wobei Z Oxyethyl 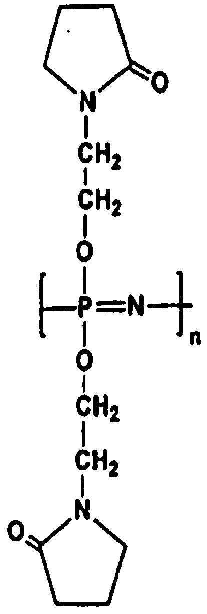 ist
oder wobei Z Oxyethyl ist und mindestens ein Teil der R-Seitengruppen Carboxylatophenoxy-Gruppen 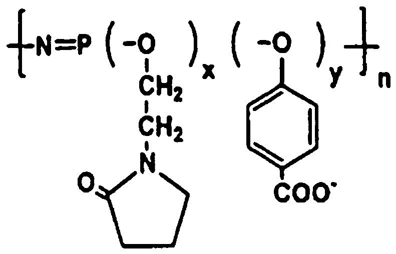 sind.

2. Polyphosphazen-Polymer nach Anspruch 1, wobei mindestens eine R-Gruppe ausgewählt ist aus der Gruppe bestehend aus Aliphat; Aryl; Aralkyl; Alkaryl; Carbonsäure; Heteroaromat; Kohlehydrate, einschließlich Glukose; Heteroalkyl; Halogen; (Aliphat)amino-, einschließlich Alkylamino-; Heteroaralkyl; Di(Aliphat)amino-, einschließlich Dialkylamino-, Arylamino-, Diarylamino-, Alkylarylamino-; -oxyaryl, einschließlich, jedoch nicht beschränkt auf -oxyphenyl-CO₂H, -oxyphenyl-SO₃H, -oxyphenylhydroxyl und -oxyphenyl-PO₃H; -oxyaliphat, einschließlich -oxyalkyl, -oxy(Aliphat)CO₂H, -oxy(Aliphat)SO₃H, -oxy(Aliphat)PO₃H, und -oxy(Aliphat)hydroxyl, einschließlich Oxy (alkyl)hydroxyl; -oxyalkaryl, -oxyaralkyl; -thioaryl; Aminosäure, Aminosäureester; Thioaliphat, einschließlich -thioalkyl; -thioalkaryl; thioaralkyl; -NHC (O)O-(Aryl oder Aliphat); -O-[(CH₂)ₓO]_{y}-CH₂)-O-[(CH₂)ₓO]_{y}(CH₂)ₓNH(CH₂)ₓSO₃H; und -O-[(CH₂)ₓO])_{y}-(Aryl oder Aliphat); wobei x 1-8 ist und y eine ganze Zahl von 1 bis 20 ist.

3. Polyphosphazen-Polymer nach Anspruch 1, welches ein Molekulargewicht von 1.000 g/mol bis 10.000.000 g/mol aufweist.

4. Polyphosphazen-Polymer nach Anspruch 1 in Form eines Homopolymers.

5. Polyphosphazen-Polymer nach Anspruch 1 in Form eines Copolymers.

6. Polyphosphazen-Polymer nach Anspruch 1, wobei n von 10 bis 10.000 ist.

7. Polyphosphazen-Copolymer nach Anspruch 5, wobei die Seitengruppen, die eine Pyrrolidon-Funktionalität enthalten, zwischen 0,5 % und 99,5 % der gesamten Seitengruppen umfassen.

8. Polyphosphazen-Copolymer nach Anspruch 7, wobei die Seitengruppen, die eine Pyrrolidon-Funktionalität enthalten, mindestens 10 % der gesamten Seitengruppen umfassen.

9. Pharmazeutische Zusammensetzung, die einen pharmazeutischen Wirkstoff und einen Träger zum Vorsehen einer kontrollierten Freisetzung des pharmazeutischen Wirkstoffes umfasst, wobei der Träger das Polyphosphazen-Polymer nach Anspruch 1 umfasst.

10. Pharmazeutische Zusammensetzung nach Anspruch 9, wobei der Träger in einer Form ausgewählt aus der Gruppe bestehend aus Matrizen, Mikrosphären, Mikrokapseln, Mizellen und stabilisierten Liposomen vorliegt.

11. Pharmazeutische Zusammensetzung nach Anspruch 9, wobei das Polyphosphazen-Polymer etwa 1 Gew.% bis etwa 99 Gew.% des Trägers zum Vorsehen der kontrollierten Freisetzung des pharmazeutischen Wirkstoffes umfasst.

12. Pharmazeutische Zusammensetzung nach Anspruch 11, wobei das Polyphosphazen-Polymer etwa 5 Gew.% bis etwa 99 Gew.% des Trägers zum Vorsehen der kontrollierten Freisetzung des pharmazeutischen Wirkstoffes umfasst.

13. Pharmazeutische Zusammensetzung nach Anspruch 9, wobei der pharmazeutische Wirkstoff ausgewählt ist aus der Gruppe bestehend aus Allergenen, Amöbiziden und Trichomonaziden, Aminosäure-Präparaten, Analeptika, Analgetika, Analgetika/Antazida, Anästhetika, Anorexika, Antazida, Antihelmintika, Antialkohol-Präparaten, Antiarthritika, Antiasthma-Mitteln, Antibakteria und Antiseptika, Antibiotika, antiviralen Antibiotika, Antikrebs-Präparaten, Anticholinergika-Inhibitoren, Antikoagulanzien, Antikonvulsiva, Antidepressiva, Antidiabetika, Antidiarrhoika, Antidiuretika, Antienuretika, Antifibrinolytika, Antifibrotika (systemisch), Antiflatulenzmitteln, Antimykotika, Antigonadotropin, Antihistaminen, Antihyperammonämiemitteln, Antiphlogistika, Antimalaria-Mitteln, Antimetaboliten, Antimigräne-Präparaten, Mitteln gegen Übelkeit, Antineoplastika, Anti-Obesitas-Präparaten, Antiparasitika, Anti-Parkinson-Arzneimitteln, Antipruritika, Antipyretika, Antispasmodika und Anticholinergika, Antitoxoplasmose-Mitteln, Antitussiva, Antivertigo-Mitteln, antiviralen Mitteln, Biologika, Wismut-Präparaten, Knochenstoffwechsel-Regulatoren, Abführmitteln, Bronchialdilatatoren, Calcium-Präparaten, kardiovaskulären Präparaten, Stimulanzien des zentralen Nervensystems, Cerumenolytika, Chelatbildnern, Choleretika, Cholesterin-Senkern und Lipid-Senkern, Koloninhalt-Säuerungsmitteln, Husten- und Erkältungspräparaten, Dekongestiva, Schleimlösern und Kombinationen, Diuretika, Emetika, Enzymen und Digestiva, Fruchtbarkeitsmitteln, Fluor-Präparaten, galaktokinetischen Mitteln, Geriatrika, Germiziden, Haematinika, Hämorrhoiden-Präparaten, Histamin-H-Rezeptor-Antagonisten, Hormonen, Hydrocholeretika, hyperglykämischen Mitteln, Hypnotika, Immunsuppressiva, Laxativa, Mukolytika, Muskelrelaxantien, narkotischen Antagonisten, narkotischen Detoxifizierungsmitteln, opthalmologischen osmotischen Dehydrierungsmitteln, otischen Präparaten, Wehenmitteln, Parasympatholytika, Nebenschilddrüsen-Präparaten, Pedikuloziden, Phosphor-Präparaten, Prämenstrual-Therapeutika, Psychostimulanzien, Chinidinen, Radiopharmazeutika, Atmungsstimulanzien, Salzsubstituten, Skabiziden, Sklerosierungsmitteln, Sedativa, Sympatholytika, Sympathomimetika, Thrombolytika, Schilddrüsen-Präparaten, Beruhigungsmitteln, Tuberkulose-Präparaten, Urikosurika, Urin-Säuerungsmitteln, Urin-Alkalisierungsmitteln, Harnwegsanalgetika, urologischen Befeuchtungsmitteln, Uteruskontraktionsmitteln, vaginalen Therapeutika und Vitaminen.

14. Poly{di[2-(2-oxo-1-pyrrolidinyl)ethoxy]phosphazen} wie durch Formel I dargestellt: 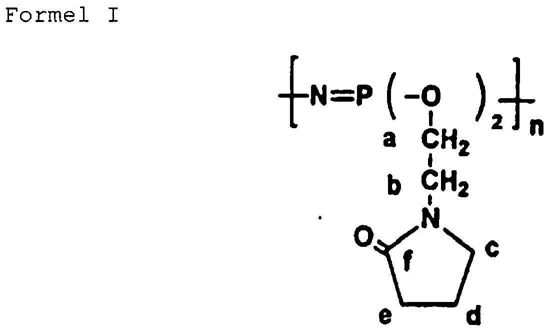 wobei jedes aus a, b, c, d, e und f NMR-Daten sind, welche das Poly{di[2-(2-oxo-1-pyrrolidinyl)ethoxy]phosphazen} wie Folgt kennzeichnen:
| NMR, ppm | |
|---|---|
| ¹H | 2,02 (**d**); 2,38 **(e)**; 3,49 (**b**); 3,51 (**c**); 4,03 (**a**) |
| ¹³C | 178,11 (**f**); 63,65 (**a**); 48,61 (**b**); 43,11 (**c**); 30,79 (**e**); 17,78 (**d**) |
| ³¹P | -7,4 |

15. Extrakoporale Vorrichtung zur Verwendung bei einer Operation, welche Beschichtungen oder Materialien umfasst, die das Polyphosphazen-Polymer nach Anspruch 1 umfassen.

16. Extrakoporale Vorrichtung nach Anspruch 15 in einer Form ausgewählt aus der Gruppe bestehend aus Blut-Oxygenatoren, Blutpumpen, Blutsensoren und Schlauchmaterial verwendet zum Transport von Blut.

17. Chirurgisch implantierbare Vorrichtung, welche Beschichtungen oder Materialien umfasst, die das Polyphosphazen-Polymer nach Anspruch 1 umfassen.

18. Chirurgisch implantierbare Vorrichtung nach Anspruch 17 in einer Form ausgewählt aus der Gruppe bestehend aus vaskulären Transplantaten, Stents, elektrischen Stimulationsleitungen, Herzklappen, orthopädischen Vorrichtungen, Kathetern, Führungsdrähten, Shunts, Sensoren, Ersatzvorrichtungen für den Nucleus pulposus, Cochlea- oder Mittelohr-Implantaten und Intraokularlinsen.

## Revendications

1. Polymère de polyphosphazène comprenant les motifs suivantes : 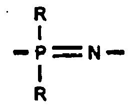 dans lesquels au moins un R répond à la formule : 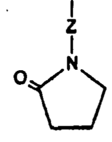 dans laquelle Z est un groupe oxyéthyle : 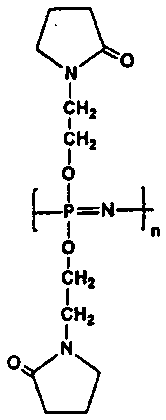 ou dans laquelle Z est un groupe oxyéthyle et au moins une partie des groupes latéraux de R sont des groupes carboxylatophénoxy :

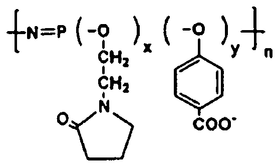

2. Polymère de polyphosphazène selon la revendication 1, dans lequel au moins un groupe R est choisi dans le groupe constitué par : un groupe aliphatique ; aryle ; aralkyle ; alkaryle ; acide carboxylique ; hétéroaromatique ; hydrates de carbone, y compris le glucose ; hétéroalkyle ; halogéno ; (aliphatique)amino- y compris alkylamino- ; hétéroaralkyle ; di(aliphatique)amino- y compris dialkylamino-, arylamino-, diarylamino-, alkylarylamino- ; -oxyaryle y compris, mais sans y être limité, -oxyphényl-CO₂H, -oxyphényl-SO₃H, -oxyphénylhydroxyle et -oxyphényl-PO₃H ; -oxyaliphatique y compris -oxyalkyle, -oxy(aliphatique)-CO₂H, -oxy(aliphatique)-SO₃H, -oxy(aliphatique)-PO₃H et -oxy(aliphatique)-hydroxyle, y compris oxy(alkyl)hydroxyle ; -oxyalkaryle, -oxyaralkyle ; -thioaryle ; acide aminé, ester d'acide aminé, thioaliphatique y compris -thioalkyle ; -thioalkaryle ; -thioaralkyle ; -NHC(O)O-(aryle ou aliphatique) ; --O--[(CH₂)ₓO]_{y}--CH₂)-O-[(CH₂)ₓO]_{y}(CH₂)ₓNH(CH₂)ₓSO₃H ; et --O--[(CH₂)_{xO}]_{y}-(aryle ou aliphatique) ; dans lesquels x vaut 1 à 8 et y est un nombre entier de 1 à 20.

3. Polymère de polyphosphazène selon la revendication 1, ayant une masse moléculaire de 1000 g/mol à 10 000 000 g/mol.

4. Polymère de polyphosphazène selon la revendication 1, sous la forme d'un homopolymère.

5. Polymère de polyphosphazène selon la revendication 1, sous la forme d'un copolymère.

6. Polymère de polyphosphazène selon la revendication 1, dans lequel n vaut de 10 à 10 000.

7. Polymère de polyphosphazène selon la revendication 5, dans lequel les groupes latéraux qui contiennent une fonctionnalité pyrrolidone constituent de 0,5 % à 99,5 % des groupes latéraux totaux.

8. Polymère de polyphosphazène selon la revendication 7, dans lequel les groupes latéraux qui contiennent une fonctionnalité pyrrolidone constituent au moins 10 % des groupes latéraux totaux.

9. Composition pharmaceutique comprenant un agent pharmaceutiquement actif et un véhicule pour fournir une libération maitrisée dudit agent pharmaceutiquement actif, ledit véhicule comprenant le polymère de polyphosphazène selon la revendication 1.

10. Composition pharmaceutique selon la revendication 9, dans laquelle ledit véhicule est sous une forme choisie dans le groupe constitué par : les matrices, les microsphères, les microcapsules, les micelles et les liposomes stabilisés.

11. Composition pharmaceutique selon la revendication 9, dans laquelle ledit polymère de polyphosphazène comprend d'environ 1 % en poids à environ 99 % en poids dudit véhicule pour fournir une libération maitrisée dudit agent pharmaceutiquement actif.

12. Composition pharmaceutique selon la revendication 11, dans laquelle ledit polymère de polyphosphazène comprend d'environ 5 % en poids à environ 99 % en poids dudit véhicule pour fournir une libération maitrisée dudit agent pharmaceutiquement actif.

13. Composition pharmaceutique selon la revendication 9, dans laquelle ledit agent pharmaceutiquement actif est choisi dans le groupe constitué par : les allergènes, les amébicides et les trichomonacides, les préparations d'acides aminés, les agents analeptiques, les analgésiques, les analgésiques/ antiacides, les anesthésiques, les anorexiques, les antiacides, les composés antihelmintiques, les préparations antialcooliques, les agents anti-arthritiques, les agents antiasthmatiques, les antibactériens et les antiseptiques, les antibiotiques, les antibiotiques antiviraux, les préparations anticancéreuses, les inhibiteurs de médicaments anticholinergiques, les anticoagulants, les anti-convulsifs, les antidépresseurs, les agents anti-diabétiques, les antidiarrhéiques, les antidiurétiques, les agents antiénurésiques, les agents anti-fibrinolytiques, les antifibrotiques (systémiques), les agents anti-ballonnements, les agents antifongiques, l'antigonadotropine, les agents antihistaminiques, les agents antihyperammonémie, les agents anti-inflammatoires, les agents antipaludéens, les agents antimétabolites, les préparation antimigraineuses, les agents antinauséeux, les antinéoplasiques, les préparations anti-obésités, les agents antiparasitaires, les médicaments antiparkinsoniens, les agents antiprurigineux, les agents antipyrétiques, les antispasmodiques et les agents anticholinergiques, les agents antitoxoplasmose, les antitussifs, les agents antivertigineux, les agents antiviraux, les agents biologiques, les préparations contre le bismuth, les régulateurs du métabolisme des os, les laxatifs intestinaux, les dilatateurs bronchiques, les préparations à base de calcium, les préparations cardiovasculaires, les stimulants du système nerveux central, les agents céruménolytiques, les agents chélatants, les agents cholérétiques, les réducteurs de cholestérol et les agent anti-hyperlipémiques, les acidifiants du contenu colique, les préparation de lutte contre la toux et le rhume, les décongestants, les expectorants et les associations, les diurétiques, les agents émétiques, les enzymes et les agents digestifs, les agents de fertilité, les préparations à base de fluor, les agents galactocinétiques, les agents gériatriques, les germicides, les agents hématiniques, les préparations hémorroïdales, les antagonistes du récepteur de l'histamine H, les hormones, les agents hydrocholérétiques, les agents hyperglycémiques, les hypnotiques, les immunosuppresseurs, les laxatifs, les agents mucolytiques, les relaxants musculaires, les antagonistes de composés narcotiques, les agents de détoxification de composés narcotiques, les agents déshydratants ophtalmologiques osmotiques, les préparations auriculaires, les ocytoxiques, les agents parasympatholytiques, les préparations parathyroïdiennes, les agents pédiculicides, les préparations à base de phosphore, les agents thérapeutiques prémenstruels, les psychostimulants, les quinidines, les agents radiopharmaceutiques, les stimulants respiratoires, les substituts de sels, les scabicides, les agents sclérosants, les sédatifs, les agents sympatholytiques, les agents sympathomimétiques, les agents thrombolytiques, les préparations thyroïdiennes, les tranquillisants, les préparation de lutte contre la tuberculose, les agents uricosuriques, les acidifiants urinaires, les agents d'alcalinisation urinaires, les analgésiques de la voie urinaire, les agents irrigants urologiques, les contractants utérins, les agents thérapeutiques vaginaux et les vitamines.

14. Poly{di[2-(2-oxo-1-pyrrolidinyl)éthoxy]-phosphazène} décrit par la formule I : 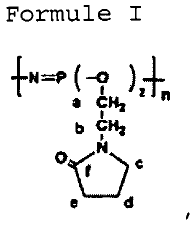 dans laquelle chacun de a, b, c, d, e et f sont des données de RMN caractérisant ledit poly{di[2-(2-oxo-1-pyrrolidinyl)éthoxy]-phosphazène} de la manière suivants :
| RMN, ppm | |
|---|---|
| ¹H | 2,02 (d) ; 2,38 (e) ; 3,49 (b) ; 3,51 (c) ; 4,03 (a) |
| ¹³C | 178,11 (f) ; 63,65 (a) ; 48,61 (b) ; 43,11 (c) ; 30,79 (e) ; 17,78 (d) |
| ³¹P | -7,4 |

15. Dispositif extracorporel destiné à une utilisation en chirurgie, comprenant des revêtements ou des matériaux comprenant le polymère de polyphosphazène selon la revendication 1.

16. Dispositif extracorporel selon la revendication 15, sous une forme choisie dans le groupe constitué par : les oxygénateurs sanguins, les pompes à sang, les capteurs de sang et les tubulures utilisées pour transporter le sang.

17. Dispositif implantable par voie chirurgicale, comprenant des revêtements ou des matériaux comprenant le polymère de polyphosphazène selon la revendication 1.

18. Dispositif implantable par voie chirurgicale selon la revendication 17, sous une forme choisie dans le groupe constitué par : les greffes vasculaires, les stents, les fils de stimulation électrique, les valves cardiaques, les dispositifs orthopédiques, les cathéters, les fils guides, les pontages, les capteurs, les dispositifs de remplacement pour la pulpe nucléaire, les implants cochléaires ou de l'oreille moyenne, et les lentilles intraoculaires.
